# EUROPEAN PATENT SPECIFICATION

(11) **EP 3 256 216 B1**
(45) Date of publication and mention of the grant of the patent: **06.09.2023**
(21) Application number: 16750064.4
(22) Date of filing: 16.02.2016
(51) Int. Cl.: A61P 9/10, A61M 11/02, A61K 9/12, A61K 31/444, A61K 47/12

(54) **MILRINONE COMPOSITION AND METHOD FOR ADMINISTERING SAME**
MILRINONZUSAMMENSETZUNG UND VERFAHREN ZUR VERABREICHUNG DAVON
COMPOSITION DE MILRINONE ET SON PROCÉDÉ D'ADMINISTRATION

(30) Priority: 13.02.2015 US 201562115798 P
(43) Date of publication of application: 20.12.2017
(73) Proprietor: Vanderbilt University, Nashville, TN 37240 (US); Cumberland Pharmaceuticals, Inc., Nashville, TN 37203 (US)
(72) Inventor: HAGLUND, Nicholas A., Westwood, Kansas 66205 (US); COX, Zachary, Nashville, Tennessee 37221 (US); PAVLIV, Leo, Cary, North Carolina 27519 (US); VILA, Andrew, Nashville, Tennessee 37221 (US)
(74) Representative: Kransell & Wennborg KB
(86) International application number: PCT/US2016/018055
(87) International publication number: WO 2016/131051

(56) References cited:
- WO-A1-2008/005053
- US-A1- 2005 049 174
- US-A1- 2005 049 174
- US-A1- 2006 030 611
- US-A1- 2006 140 873
- US-A1- 2011 008 325
- US-B2- 7 348 362

## Description

### TECHNICAL FIELD

The presently-disclosed subject matter relates to milrinone compositions and methods for making and using the same. In particular, embodiments of the presently-disclosed subject matter relate to nebulized inhaled milrinone compositions for use in methods for treating heart failure.

### INTRODUCTION

Approximately 5.7 million Americans suffer from heart failure, a leading cause of both morbidity and mortality in the United States. Heart failure was listed as a contributing cause in more than 280,000 deaths in 2008 in the U.S. (1 in 9) and about half of patients diagnosed with heart failure die within 5 years. Patients with end stage (Stage D) heart failure have significant symptoms (including fatigue and dyspnea) which prevent them from being able to perform most activities of daily life. These patients often require repeated or prolonged hospitalizations for disease management, which contributes significantly to the cost of heart failure for the United States (34.4 billion each year).

Milrinone, a phosphodiesterase III inhibitor, is one of the inotropic medications that has been studied and used in the treatment of acutely decompensated heart failure. Several studies have evaluated chronic intravenous (IV) inotrope use in end stage (Stage D) heart failure for palliation of symptoms as well as evaluated effect on cost through decreased hospital readmissions. Initiation of inotropes including milrinone can decrease hospital costs by reducing hospitalization length and readmissions rates. However, current milrinone compositions and administration methods can cause severe complications, serious arrhythmias, or hypotensive events. The concern with current milrinone use is the possibility of increased mortality associated with therapy despite improved symptoms and hemodynamics (i.e., increased cardiac output, decreased filling pressures). This has been documented with chronic use of oral inotropes.

The use of "milrinone" herein includes pharmaceutically acceptable salts, and pharmaceutically acceptable derivatives. As used herein, pharmaceutically acceptable derivative refers to a compound having a structure derived from the structure of milrinone and whose structure is sufficiently similar to those disclosed herein and based upon that similarity, would be expected by one skilled in the art to exhibit the same or similar activities and utilities as the claimed compounds, or to induce, as a precursor, the same or similar activities and utilities as the claimed compounds. Exemplary derivatives include salts, esters, amides, salts of esters or amides, and N-oxides of milrinone. One example is milrinone lactate.

PRIMACOR^{™} is a brand of milrinone lactate, designated chemically as 1,6-dyhydro-2-methyl-6-oxo-[3,4'-bipyridine]-5-carbonitrile lactate and has the following structure:

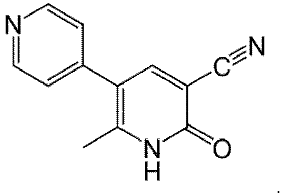

U.S. 2005/0049174 relates to facilitating weaning, reducing morbidity, and reducing mortality in cardiac surgeries involving extra-corporal circulation. According to '174, prophylactic strategies aimed at delivering vasodilators through inhalation in the pulmonary tree treat and prevent right ventricular dysfunction by reducing right ventricular afterload, facilitate separation from bypass and consequently decrease hemodynamic complications, morbidity and mortality. Examples of suitable vasodilators include milrinone (PRIMACOR^{™}).

A method of making milrinone is disclosed in US Patent 4,413,127:

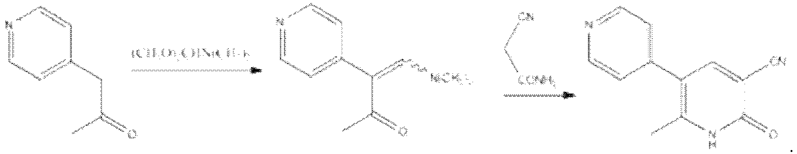

PRIMACOR^{™} is administered by injection. However, the use of intravenous milrinone has been reported as increasing serious atrial and ventricular arrhythmias in patients with New York Heart Association (NYHA) class III-IV ischemic heart failure without hemodynamic compromise. Milrinone is commonly delivered by a chronically indwelling peripherally inserted central catheter (PICC), which is prone to infection and clot formation. PICC complications, independent of the complications of IV milrinone itself, result in hospitalizations in 27% of patients utilizing a PICC for IV milrinone delivery. Current intravenous milrinone administration methods can also result in supratherapeutic plasma levels of milrinone that are outside the accepted therapeutic range and could increase milrinone adverse events without adding additional benefit to the patient. For these reasons, the American Heart Association/American College of Cardiology (AHA/ACC) practice guidelines recommend use of intravenous milrinone only for patients presenting clinical evidence of hypotension associated with hypo-perfusion and elevated cardiac filling pressures in order to maintain systemic perfusion and preserve end-organ performance.

Hence, there remains a need for novel milrinone compositions and methods for administering the same that improve hemodynamics without the above-described shortcomings of the drug itself and its delivery method.

### BRIEF DESCRIPTION OF THE FIGURE

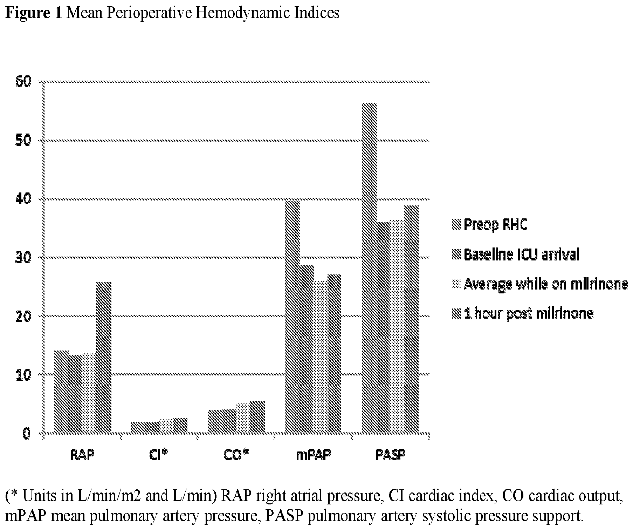

Figure 1 is a graph that shows mean perioperative hemodynamic indices. See Example 1.

### DETAILED DESCRIPTION OF EXEMPLARY EMBODIMENTS

The details of one or more embodiments of the presently-disclosed subject matter are set forth in this document. Modifications to embodiments described in this document, and other embodiments, will be evident to those of ordinary skill in the art after a study of the information provided in this document. The information provided in this document, and particularly the specific details of the described exemplary embodiments, is provided primarily for clearness of understanding and no unnecessary limitations are to be understood therefrom. In case of conflict, the specification of this document, including definitions, will control.

The presently-disclosed subject matter relates to pharmaceutical formulations comprising milrinone for use in a method for treating heart failure as defined in claims 1 and 7, and in the claims dependent therefrom.

The term "nebulized" refers to a composition that has been transformed into a medical aerosol, optionally with ambient air, and which can be inhaled by a subject inhaling through a mouthpiece associated with a "nebulizer" (also referred to as inhaler herein).

The term "aerosol" describes a nebulized liquid preparation consisting of fine particles carried by a gas, usually air, to the site of therapeutic action. or delivery target.

The terms "treatment" or "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term can include palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

The term "administering" refers to any method of providing a composition and/or pharmaceutical composition thereof to a subject. In the context of the present nebulized compositions, "administering" and the like can include administration by inhalation, nasal administration, and the like. Administration can be continuous or intermittent. In various aspects, a preparation can be administered therapeutically; that is, administered to treat an existing disease or condition (e.g., heart failure). In further various aspects, a preparation can be administered prophylactically; that is, administered for prevention of a disease or condition.

The term "subject" is used herein to refer to a target of administration, which optionally displays symptoms related to a particular disease, pathological condition, disorder, or the like. The subject of the herein disclosed methods can be a vertebrate, such as a mammal, a fish, a bird, a reptile, or an amphibian. Thus, the subject of the herein disclosed methods can be a human, non-human primate, horse, pig, rabbit, dog, sheep, goat, cow, cat, guinea pig, or rodent. The term does not denote a particular age or sex. The term "subject" includes human and veterinary subjects. The term "subject" may be used interchangeable with the term "patient" herein.

The term "therapeutically effective amount" refers to the amount of the compound or pharmaceutical composition that will elicit the biological or medical response of a tissue, system, animal or human that is being sought by the researcher, veterinarian, medical doctor or other clinician, e.g., restoration or maintenance of eyesight and/or amelioration of a retinal vein occlusion.

The "pharmaceutically acceptable" refers to the fact that the carrier, diluent or excipient must be compatible with the other ingredients of the formulation and not deleterious to the recipient thereof.

The present milrinone compositions can be aqueous compositions. The water in embodiments of the present milrinone compositions can include sterile water. In some embodiments the compositions also include one or more other substances.

The present milrinone compositions may be salt-free.

The present compositions can include a range of milrinone compositions. Some embodiments of the present compositions include about 0.1 mg/ml to about 10.0 mg/ml of milrinone, about 0.1 mg/ml to about 5.0 mg/ml of milrinone, or about 0.5 mg/ml to about 2.5 mg/ml of milrinone. Certain embodiments can include about 0.1 mg/ml, 0.2 mg/ml, 0.3 mg/ml, 0.4 mg/ml, 0.5 mg/ml, 0.6 mg/ml, 0.7 mg/ml, 0.8 mg/ml, or 0.9 mg/ml of milrinone. Other embodiments can include about 1.0 mg/ml, 2.0 mg/ml, 3.0 mg/ml, 4.0 mg/ml, 5.0 mg/ml, 6.0 mg/ml, 7.0 mg/ml, 8.0 mg/ml, 9.0 mg/ml, 10.0 mg/ml of milrinone, or any range in-between. Other embodiments can include about 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, or any range in-between. In embodiments of the present invention, the amount is about 10 mg/ml. In others, the amount is about 10 to about 15 mg/ml.

The pH of the compositions of the present can vary. For example, the pH can vary from about 2.6 to about 3.4. Others range from about 3 to about 3.2.

In some embodiments the other substances that can enhance the nebulizing quality of the compositions. For instance, in some embodiments substances selected from potassium chloride, sodium chloride, lactic acid, sodium lactate, citric acid, sodium citrate, phosphoric acid, mono, di, and tri basic sodium phosphate, acetic acid, sodium acetate, hydrochloric acid, sodium hydroxide, triacetin, monoacetin, propylene glycol, urea, sorbitol, dextrose, glycerol, and combinations thereof can be included in the compositions, and, without being bound by theory or mechanisms, such substances may increase the nebulization efficiency of the compositions. In other embodiments, the nebulizing enhancing substances, or excipient is lactic acid.

In this regard, the present compositions can include a range of excipients in addition to water and milrinone. Some embodiments of milrinone compositions comprise about 1 wt%, 2wt%, 3wt%, 4wt%, 5 wt%, 6 wt%, 7 wt%, 8 wt%, 9 wt%, or 10 wt%, 11 wt%, 12 wt%, 13 wt%, 14 wt%, 15 wt%, or 16 wt% (and any range in between) of another substance/excipient (e.g., lactic acid) in solution with the milrinone and water.

The compositions of the present invention may further comprise surfactants, particularly in compositions that are used in connection with metered dose inhalers. The surfactant of the pharmaceutical composition according to the invention can be chosen among different classes of surfactants of pharmaceutical use. Surfactants suitable to be used in the present invention are all those substances characterized by medium or low molecular weight that contain a hydrophobic moiety, generally readily soluble in an organic solvent but weakly soluble or insoluble in water, and a hydrophilic (or polar) moiety, weakly soluble or insoluble in an organic solvent but readily soluble in water. Surfactants are classified according to their polar moiety. Therefore, surfactants with a negatively charged polar moiety are called anionic surfactants, while cationic surfactants have a positively charged polar moiety. Uncharged surfactants are generally called nonionic, while surfactants charged both positively and negatively are called zwitterionic. Examples of anionic surfactants are salts of fatty acids (better known as soaps), sulphates, sulphate ethers and phosphate esters. Cationic surfactants are frequently based on polar groups containing amino groups. Most common nonionic surfactants are based on polar groups containing oligo- (ethylene-oxide) groups. Zwitterionic surfactants are generally characterized by a polar group formed by a quaternary amine and a sulfuric or carboxylic group. Specific examples of this application are the following surfactants: benzalkonium chloride, cetrimide, docusate sodium, glyceryl monolaurate, sorbitan esters, sodium lauryl sulphate, polysorbates, phospholipids, biliary salts.

Specific examples of this application are the following surfactants: benzalkonium chloride, cetrimide, docusate sodium, glyceryl monolaurate, sorbitan esters, sodium lauryl sulphate, polysorbates, phospholipids, biliary salts.

Nonionic surfactants, such as polysorbates and polyethylene and polyoxypropylene block copolymers, known as "Poloxamers", are preferred. Polysorbates are described in the CTFA International Cosmetic Ingredient Dictionary as mixtures of sorbitol and sorbitol anhydride fatty acid esters condensed with ethylene oxide. Particularly preferred are nonionic surfactants of the series known as "Tween", in particular the surfactant known as "Tween 80", a polyoxyethylen sorbitan monolaurate available on the market.

The presence of a surfactant, and preferably of Tween 80, is necessary to reduce electrostatic charges found in formulations without it, the flow of the powder and the maintenance of the solid state in a homogeneous way without initial crystallization.

As indicated above, nebulizers, in which the drug is dissolved or dispersed in suspension form and delivered in the lung as fine nebulized particles are used with embodiments of the present invention.

Additionally, dry powder inhalers, capable of delivering the drug loaded in the device as dry micronized particles are used with embodiments of the present invention.

Further, pressurized inhalers/metered dose inhalers, for which the drug - again in the form of small solution or suspension droplets - is delivered to the lower pulmonary region by an inert gas rapidly expanded in the air from a pressurized device, are used with embodiments of the present invention.

Non-pressurized, soft mist inhalers may also be used with embodiments of the present invention, as can pressurized inhalers that contain compositions of the present invention in addition to a propellant.

In an embodiment, a dry powder formulation associated with such inhalers can be suspended in non-aqueous liquids, for example, chlorofluorocarbons, hydrofluoroalkanes, hydrochlorofluorocarbons, and/or fluorocarbons, which may be used in pressurized metered dose inhalers or aerosolized as dry powder in active or passive inhalers. Included as an embodiment of the present invention includes an air-tight container with a milrinone composition of the present invention and a propellant.

The presently-disclosed subject matter also relates to systems for administering a nebulized milrinone composition. As used herein, a nebulized composition or solution refers to a composition that is dispersed in air to form an aerosol. Thus, a nebulized composition is a particular form of an aerosol.

As used herein, a nebulizer is an instrument that is capable of generating very fine liquid droplet for inhalation into the lung. Within this instrument, the nebulizing liquid or solution is atomized into a mist of droplets with a broad size distribution by methods known to those of skill in the art, including, but not limited to, compressed air, ultrasonic waves, or a vibrating orifice. Nebulizers may further contain, e.g., a baffle which, along with the housing of the instrument, selectively removes large droplets from the mist by impaction. Thus, the mist inhaled into the lung contains fine aerosol droplets.

In some embodiments the system is comprised of a nebulizer capable of housing and administering a milrinone composition. Exemplary nebulizers include, but are not limited to, those described in U.S. Patent Nos. 5,758,637, 6,983,747, 7,059,320, 7,322,349.

In some embodiments the nebulizer is a jet nebulizer. The term "jet nebulizer" refers to devices that feed compressed gas (e.g., air, oxygen, etc.) into a nozzle to create a reduction of static pressure which draws the milrinone composition from a reservoir. The milrinone composition that is drawn up is broken up by the gas jet into a dispersion of droplets. A portion of these droplets are then released from the nozzle as an aerosol that can be inhaled.

In other embodiments the nebulizer includes an ultrasonic nebulizer. The term "ultrasonic nebulizer" refers to a type of device that utilizes ultrasonic energy to create a fine mist (i.e., aerosol) that can be inhaled by a subject. In some instances, an ultrasonic nebulizer operates by focusing a beam of ultrasonic energy on to a composition in order to nebulize the composition. In other instances, an ultrasonic nebulizer can include an ultrasonically vibrating plate, wherein a composition can be nebulized by placing the composition on to the plate.

In other embodiments, the nebulizer is a vibrating mesh nebulizer. The term "vibrating mesh nebulizer" refers to devices that use a vibrating mesh or plate with multiple apertures to generate a fine-particle, low-velocity aerosol. In many cases, these devices have a high efficiency of delivering aerosol to the lung, such that the nominal dose of drugs to be administered could be substantially reduced. Moreover, the volume of drug solution left in these new devices when the nebulization has ceased is negligible, so there is potential to improve the cost-effectiveness of administering expensive medications. Because these devices sometimes nebulize at a faster rate than conventional jet or ultrasonic nebulizers, the duration of each treatment could potentially be shortened.

In yet other embodiments the system includes an inhaler that can administer a nebulized composition. The inhaler includes a container that comprises propellant and a composition under pressure. The inhaler further includes a housing for receiving and activating the container. When the container is engaged in the housing, the propellant pushes the composition through a nozzle, thereby forming an aerosol of the composition.

Those of ordinary skill in the art upon reviewing this application will appreciate other systems, including variations of the systems described herein, that can be utilized for nebulizing and administering the present compositions.

A system can house a range of volumes of the composition. In some embodiments the systems can house about 1 mL, 2 mL, 3 mL, 4 mL, 5 mL, 6 mL, 7 mL, 8 mL, 9 mL, 10 mL, 15 mL, 20 mL, 25 mL, 30 mL, 35 mL, 40 mL, 45 mL, 50 mL 60 mL, 70 mL, 80 mL, 90 mL, 100 mL, or more of a composition. The volume of a milrinone composition within a system can therefore be sufficient for administering one or more doses of the composition to a subject in need thereof. The volume of an individual dose can depend on, among other things, the concentration of milrinone in a composition, the disease or condition being treated, and the particular subject being treated.

Additionally, on the other hand, current methods commonly administer milrinone by a chronically indwelling peripherally inserted central catheter (PICC). PICCs are prone to infection and clot formation, can increase morbidity and hospitalization time for patients. Furthermore, current intravenous milrinone administration methods can also result in supratherapeutic plasma levels of milrinone that are outside the accepted therapeutic range and could increase milrinone adverse events without adding additional benefit to the patient. In contrast, the present nebulized milrinone compositions, when administered by inhalation, can result in relatively lower, yet therapeutic, plasma levels of milrinone. The present compositions can therefore result in less medication toxicity and fewer adverse side effects when compared to known administration methods, such as intravenous administration.

In some embodiments, the composition is for use in methods including administering by inhalation fixed amounts of the nebulized milrinone composition at regular intervals. The amount of the milrinone composition to be administered is intended to provide a nontoxic but sufficient amount of milrinone, such that the desired effect is produced. Thus, the exact amount of the milrinone composition that is required will vary from subject to subject, depending on the species, age, and general condition of the subject, the severity of the condition being treated, the particular carrier or adjuvant being used and its mode of administration, and the like. Similarly, the dosing regimen should also be adjusted to suit the individual to whom the composition is administered and will also vary with age, weight, metabolism, etc. of the individual.

In some embodiments of the composition for use, about 6 to about 24 mg is provided to a subject in each dose, including about 6, 7, 8, 9, 10, 11, 12, 13, 14, 15, 16, 17, 18, 19, 20, 21, 22, 23, or 24 mg. Furthermore, in some embodiments a subject can be administered one or more fixed doses, the doses ranging from continuous nebulization to intermittent doses each being separated by approximately 4 to 12 hours, including about 4.0, 4.5, 5.0, 5.5, 6.0, 6.5, 7.0, 7.5, 8.0, 8.5, 9.0, 9.5, 10.0, 10.5, 11.0, 11.5, or 12.0 hours. Inhalation administration avoids the need for PICC placement, reducing the risk of hospitalization or mortality from PICC-related infection or thrombosis. In an outpatient setting, inhalation administration would not require a home infusion pharmacy and home health care nurses to compound and administer the milrinone, as is necessary with IV milrinone via a PICC line. Thus, inhalation administration could reduce health care costs and utilization.

The present treatment compositions can be applied to treat a variety of diseases and conditions. In some embodiments, the disease and/or condition is pulmonary hypertension. In some embodiments the disease and/or condition being treated includes depressed myocardial function. The depressed myocardial function can be following surgery. The depressed myocardial function could be heart failure. The term "heart failure" as used herein generally refers to one or more diseases and/or conditions in which the heart no longer pumps an adequate supply of blood in relation to the venous return and the metabolic needs of the tissues of a subject at that particular moment. "Congestive heart failure" refers to that state in which abnormal fluid accumulation occurs in different parts of the body (e.g., swelling in limbs, pulmonary edema, pleural effusion) as the result of heart failure including, but not limited to, circulatory failure due to mechanical abnormalities, myocardial (muscular) failure, peripheral circulatory failure, and cardiac, and non-cardiac circulatory overload.

Heart failure can include various diseases and conditions, including, but not limited to, systolic heart failure (inability to develop enough force during systole), diastolic heart failure (inability to fully relax between beats), right-sided heart failure (failure of the right ventricle), left-sided heart failure (failure of the left ventricle), forward heart failure (inability to pump enough blood to meet the tissue demand during exercise or rest), backward heart failure (failure of the heart to supply enough blood to meet tissue demand when ventricular filling pressures are high), and high-output heart failure (cardiac output insufficient to meet an excessively high tissue demand).

The New York Heart Association has developed a heart condition classification expressed as Stages I, II, III, and IV. The stage is determined by symptoms exhibited by a subject, such as the subject's ability to breathe, exercise, or perform normal physical activities. Stage I is characterized by no breathlessness with normal physical activity. Stage II is characterized by breathlessness associated with normal physical activity. Stage III is characterized by breathlessness associated with even minimal physical activity. Stage IV is characterized by breathlessness even while a patient is at rest.

Similarly, the American College of Cardiology/American Heart Association stages patients according to the progression of their heart failure. Subjects at stage A have a high risk for developing heart failure and have one or more risk factors for developing heart failure. Subjects at stage B have asymptomatic heart failure, and this stage includes subjects who have an enlarged or dysfunctional left ventricle from any cause. Subjects at stage C have symptomatic heart failure, and experience heart failure symptoms such as shortness of breath, fatigue, inability to exercise, and the like. Lastly, subjects at stage D have a refractory end-stage heart failure and have heart failure symptoms at rest in spite of medical treatment.

Heart failure can also include acute decompensated heart failure. The term "acute decompensated heart failure" as used herein generally refers to new or worsening symptoms or signs of dyspnea, fatigue, or edema that lead to hospital admission or unscheduled medical care. These symptoms are consistent with an underlying worsening of left ventricular function. The term "acute" in relation to heart failure can refer to symptoms of heart failure that are presented in a subject with no prior history of heart failure. "Acute" heart failure can also occur for subjects having previous myocardial dysfunction, such as congestive heart failure (CHF) patients who suddenly present new or worsening symptoms.

Heart failure, as discussed herein, can be classified as non-ambulatory or ambulatory. Furthermore, heart failure can be inclusive of "chronic heart failure" as the term is used in the art. Those of ordinary skill in the art will further recognize the scope of diseases and conditions that are classified as heart failure.

In particular embodiments, the compositions are for use in a method for treating ambulatory heart failure subjects. In some embodiments the compositions are for use in a method for treating ambulatory stage D heart failure subjects. For ambulatory and/or non-ambulatory stage D heart failure, as well as other heart failure, administration of milrinone may be required by a subject. In some instances, administration of a milrinone composition by a subject will be performed in an outpatient setting. Accordingly, the present methods of administrating a nebulized milrinone composition allow a subject to administer milrinone in by controlled, simple, and non-invasive means, particularly when compared to known intravenous administration methods.

The presently-disclosed treatment methods, not claimed, can reduce or eliminate the need for invasive procedures and the potential for over-dosing of milrinone. The presently-disclosed nebulized milrinone treatment methods can therefore minimize or eliminate complications, costs, and hospital readmission rates in subjects being treated for heart failure with milrinone. For instance, relative to known methods, the present nebulized compositions and methods can produce relatively lower serum plasma milrinone levels that are, in some instances consistently, within a therapeutic range, thereby resulting in less systemic adverse effects when compared to "standard of care" fixed dose intravenous milrinone therapy.

Regarding the compositions for use in nebulized milrinone treatment methods, said methods can include continuously administering the nebulized composition to a subject. Said nebulized milrinone treatment methods include administering the nebulized composition to a subject at regular and/or preset intervals. Regarding the compositions for use in nebulized milrinone treatment methods, said methods including administering the nebulized composition to a subject on an as-needed basis. Indeed, the present nebulized compositions are particularly easy to use on an as-needed basis relative to intravenous means that are highly invasive. Thus, the present compositions for use in treatment methods are highly adaptable for administering milrinone in various different setting and for treating various different forms of heart failure.

### EXAMPLES

The presently-disclosed subject matter is further illustrated by the following specific but non-limiting examples. Some examples are prophetic. Some of the following examples may include compilations of data that are representative of data gathered at various times during the course of development and experimentation related to the presently-disclosed subject matter.

### Example 1

This Example represents a study that describes benefits of the present invention, including the tolerability, feasibility, efficacy and pharmacokinetics of inhaled milrinone (iMil) delivery after CF-LVAD implantation.

**Methods.** The present inventors evaluated fixed dose nebulized iMil delivered into a ventilator circuit for 24 hours in 10 post-operative CF-LVAD (HeartMate-II=10) patients. Tolerability (arrhythmias, hypotension and hypersensitivity reaction), efficacy (hemodynamic), pharmacokinetics (plasma milrinone levels) and cost data were collected.

**Results.** Mean age was 56±9 years, 90% male, mean INTERMACS profile was 2.5±0.8. No new atrial arrhythmia events occurred, however 3 (30%) ventricular tachycardia (1 non-sustained, 2 sustained) events occurred. Sustained hypotension, drug hypersensitivity, death or need for RVAD were not observed. Invasive mean pulmonary artery pressure from baseline to during iMil therapy was improved (p=0.017). Mean plasma milrinone levels (ng/ml) at baseline, 1, 4, 8, 12, and 24 hour times were 74.2±35.4, 111.3±70.9, 135.9±41.5, 205.0±86.7, 176.8±61.3 187.6±105.5 respectively. Reduced institutional cost was observed when iMil was compared to nitric oxide therapy over 24 hours ($165.29 *vs* $1,944.00 respectively).

**Conclusions.** iMil delivery after CF-LVAD implantation was well tolerated, feasible, and demonstrated favorable hemodynamic, pharmacokinetic and cost profiles.

### ABBREVIATIONS

**INTERMACS** Interagency Registry for Mechanically Assisted Circulatory Support
**RV** Right ventricle
**CF-LVAD** Continuous-flow left ventricular assist device
**RVAD** Right ventricular assist device
**MAP** Mean arterial pressure
**VT** Ventricular tachycardia
**ICU** Intensive care unit
**RAP** Right atrial pressure
**mPAP** Mean pulmonary artery pressure
**PASP** Pulmonary artery systolic pressure
**CI** Cardiac index
**CO** Cardiac output
**LVEDV** Left ventricular end diastolic volume
**LVEDd** Left ventricular end diastolic dimension
**SV** Stroke Volume
**TAPSE** Tricuspid annular plane systolic excursion
**HPLC** High liquid chromatography

### MATERIAL AND METHODS

Patients fulfilling standard criteria for CF-LVAD placement at the University of Nebraska Medical Center were recruited for enrollment between April 2012 and June 2013. The protocol was approved by the University of Nebraska Institutional Review Board. Patients deemed appropriate for bridge to transplant or destination therapy indications were consented prior to CF-LVAD implantation to receive post-operative inhaled milrinone. Ten consecutive patients were consented and received inhaled milrinone therapy. Data was collected prospectively. Exclusion criteria included pregnant or breast-feeding patients and documented allergy to milrinone. Patients were compared to institutional and national database CF-LVAD controls. All institutional controls underwent inhaled nitric oxide therapy and intravenous administration of milrinone. Patients enrolled in this study received inhaled milrinone in place of inhaled nitric oxide (40 ppm) and intravenous milrinone. All patients were converted to intravenous milrinone, if clinically indicated, after study protocol was completed.

### Study End-point Determination

The primary end point was safety, which included documentation of 1) new atrial arrhythmias, lasting greater than 30 seconds or resulting in hemodynamic instability (mean arterial pressure (MAP) <60 mmHg or requiring medical intervention) 2) sustained ventricular tachycardia (VT) lasting greater than 30 seconds, not related to an LVAD suction event or any ventricular arrhythmia resulting in hemodynamic instability (sustained hypotension with a MAP < 60mmHg not related to secondary causes such as acute blood loss or tamponade) and 3) hypersensitivity reaction to milrinone leading to systemic hypotension (MAP <60mmHg or requiring medical intervention), bronchospasm or rash 4) Death by 30 days.

Secondary end points were designed to assess the efficacy, cost effectiveness and pharmacokinetic profile of inhaled milrinone therapy. Efficacy endpoints included hospital and intensive care unit (ICU) length of stay, days on inotropes, mortality at 30 days and changes in right ventricular stroke work index (RSVWi) [13] and right heart catheterization and echocardiographic parameters from baseline values. Right heart catheterization was performed during the hospitalization before LVAD implantation and included the following parameters: right atrial pressure (RAP), mean pulmonary artery pressure (mPAP), pulmonary artery systolic pressure (PASP), cardiac index (CI) and cardiac output (CO). Echocardiographic data was collected retrospectively from echocardiograms closest to LVAD implantation date (studies performed > 1 month before surgery were not included) and prospectively obtained within 72 hours after LVAD implantation. Echocardiographic parameters included: left ventricular end diastolic volume (LVEDV), left ventricular end diastolic dimension (LVEDd), cardiac output (CO), PASP, qualitative RV function, and tricuspid annular plane systolic excursion (TAPSE).

Blood samples to determine plasma milrinone levels were obtained at baseline, 1, 4, 8, 12 and 24 hours after initiation of inhaled milrinone. Plasma milrinone levels were determined by ultraviolet high liquid chromatography (HPLC) method [14].

Equipment and medication costs of inhaled milrinone delivery were compared to inhaled nitric oxide therapy on a per patient basis calculated over the study duration (24 hours). All cost data was calculated based on based on institutional 2012 prices.

### Characterization of Inhaled Milrinone

Inhaled milrinone validation studies were performed in order to characterize inhaled milrinone aerosol particle size, evaluate nebulization degradation profile, and quantify drug delivery using a vibrating mesh nebulizer connected to a mechanical ventilator circuit. Mass spectrometry analysis of inhaled milrinone samples did not identify new drug formation or degradation products. A pharmaceutical grade impactor was used to determine median aerodynamic particle size and HPLC was used to determine the amount of milrinone collected at the distal end of the experimental endotracheal tube [15]. Based on this analysis, we prepared a solution of milrinone diluted in 0.9% saline to a final concentration of 0.5mg/ml to be delivered via continuous infusion by an Alaris^{®} infusion pump set at a fixed rate of 12ml/hr connected to an Areoneb^{®} Solo (Aerogen Ltd., Galway, Ireland) vibrating mesh nebulizer and Aeroneb^{®} Pro-X nebulizer control unit (Aerogen Ltd., Galway, Ireland). The nebulizer was connected to a mechanical ventilator (Servo-I, Maquet Inc. Wayne NJ) with a dual heated wire ventilator circuit (Teleflex Hudson RCI, Research Triangle Park, NC) proximal to the heated humidifier (Teleflex Hudson Neptune, Research Triangle Park, NC). Despite continuous nebulization, delivery of the aerosolized drug product occurred in an 'intermittent-continuous' fashion based on the mechanical ventilator respiratory rate. All inhaled milrinone product was administered by a licensed respiratory therapist.

### Clinical Delivery of Inhaled Milrinone

Delivery of inhaled milrinone was initiated five minutes before the first weaning attempt from cardiopulmonary bypass after LVAD implantation in the operating suite. Intraoperative transesophageal echocardiography was performed and analyzed by a single cardiac anesthesiologist, certified in the interpretation of intraoperative echocardiography. Prior to initiation of inhaled milrinone, blood samples were obtained and analyzed for baseline plasma levels of milrinone. Patient arrival in the ICU immediately after LVAD implantation was defined as `time zero.' Further blood samples to determine plasma milrinone levels were obtained at 1, 4, 8, 12 and 24 hours after arrival to the ICU. Hemodynamic values from indwelling pulmonary artery catheter and routine vital signs were also obtained at 1, 4, 8, 12 and 24 hour intervals. Patients received inhaled milrinone for a maximum of 24 hours or until the patient was extubated (if less than 24 hours). Final hemodynamic values were obtained 1 hour after extubation or cessation of inhaled milrinone therapy. All patients underwent a transthoracic echocardiogram 48-72 hours after cessation of inhaled milrinone.

Subjects were withdrawn from the study if they failed to withdraw completely from cardiopulmonary bypass support as defined by a MAP <60mmHg despite aggressive inotropic support (>2 vasoactive agents), required urgent hemodialysis or at the request of the cardiac surgeon/cardiac anesthesiologist.

### Statistics

Patient data collected was analyzed using PC SAS version 9.3 (Cary, NC). The statistical level of significance was set at p≤0.05. For continuous variables, the mean, standard deviation, minimum/maximum, 25th/75th percentiles, and the median are presented. For categorical variables, frequency and percentages are presented for each variable of interest. Continuous variables that had pre/post values were compared using a paired t-test. Study variables with comparable institutional standards were compared using sign rank test and chi squared test.

### RESULTS

Baseline demographic variables and pre-operative risk scores are presented in Table 1. Median preoperative 6 minute walk distance was 281 feet. Average right ventricular failure risk score (RVFRS) was 1.5 ± 2.0 [16]. Average right ventricular stroke work index was 571±218. Average preoperative CVP:PCWP ratio for the cohort was 0.54±0.04. Patients received continuous inhaled milrinone therapy for a duration of 8 (100%), 12 (78%) and 24 (67%) hours.

### Primary Outcomes

No atrial arrhythmias, sustained hypotension or hypersensitivity to inhaled milrinone were observed. Ventricular arrhythmias were observed in 1 patient with non-sustained VT and 2 patients with sustained VT. All patients included in this analysis had documented cases of either non-sustained or sustained VT prior to LVAD implant. The rate of ventricular arrhythmias during inhaled milrinone therapy (n=3, 30%) was comparable to arrhythmic events in patients (n=1919, 36%) after CF-LVAD implantation recorded in the INTERMACS data base from June 2006 to June 2012 (p=0.99). No deaths occurred while receiving inhaled milrinone therapy. Deaths in the study group (0/10) 30 days after CF-LVAD implantation were comparable to the institutional CF-LVAD 30 day death rate (12%, 12/102; p=0.60). No patients were withdrawn from inhaled milrinone therapy. Although no patients required RVAD, 1 patient required support with veno-arterial extracorporeal membrane oxygenation (ECMO) within hours of LVAD implantation, however no absolute cause was identified, and the patient completed inhaled milrinone therapy at the discretion of the implanting surgeon.

### Secondary Outcomes

Analysis of secondary outcomes revealed that median ICU and total hospital length of stay in the inhaled milrinone group (5 and 33 days respectively) were comparable to the institutional ICU and total hospital length of stay (5 and 28 days; p=0.49 and p=0.26 respectively). Median total days on inotrope support were comparable between the inhaled milrinone (9 days) and institutional (6 days) groups (p=0.08).

Detailed hemodynamic comparisons are shown in Table 2. Comparisons between preoperative mean and median RVSWi (571 and 453) to values obtained 1 hour after milrinone cessation (383 and 326) respectively were not significantly different (p=0.179). Comparison of hemodynamic measurements from preoperative right heart catheterization to time of ICU arrival, during inhaled milrinone therapy and 1 hour after cessation of inhaled milrinone, suggest early postoperative improved RV hemodynamics after CF-LVAD implantation and while on inhaled milrinone therapy (Table 2 and Figure 1). CI and CO consistently improved throughout the post-operative period. The RAP decreased while on inhaled milrinone when compared to baseline measurements, however did not reach statistical significance. Upon cessation of inhaled milrinone, the RAP increased significantly compared to baseline, ICU arrival and mean RAP values while on inhaled milrinone therapy. Echocardiographic variables (LVED volume, LVIDd, and PASP) showed significant decreases after LVAD implantation while on inhaled milrinone therapy when compared to pre-LVAD implant echocardiography variables (all p<0.009), however TAPSE and qualitative RV function were not significantly different (p=0.846 and p=0.347 respectively).

### Plasma Milrinone Levels

Plasma milrinone levels were monitored at baseline, during and after the administration of inhaled milrinone as displayed in Table 3. Inhaled milrinone therapy led to plasma milrinone levels within the accepted therapeutic milrinone range of 100-300 ng/mL [17, 18]. These data demonstrate initial increases in plasma milrinone levels (hours 0-4) with subsequent plateau effect of plasma milrinone levels (hours 4-24) with continuous medication administration.

The per patient cost of administering inhaled milrinone versus inhaled nitric oxide for 24 hours was significantly reduced (Table 4).

### DISCUSSION

This study suggests that the delivery of postoperative inhaled milrinone is feasible, well tolerated and effective for patients undergoing CF-LVAD placement. Evaluation of our primary endpoints revealed no new atrial arrhythmias, sustained hypotension, or hypersensitivity related to the administration of inhaled milrinone. Three patients did experience ventricular arrhythmias; however this was comparable to the rate of ventricular arrhythmias reported in the INTERMACS registry. One patient required veno-arterial ECMO support within hours after CF-LVAD implantation and separation from cardiopulmonary bypass, however this was not felt to be related to inhaled milrinone therapy and the patient completed the full study protocol.

### Effects of Inhaled Milrinone

No significant differences in ICU and total hospital length of stay, total days on inotropes or 30 day mortality was found when compared to institutional CF-LVAD controls who received inhaled nitric oxide. Postoperatively, in the context of CF-LVAD support, hemodynamic and echocardiographic changes were consistent with improving heart failure. These findings suggest that patients who received inhaled milrinone experienced no more RV dysfunction than would be expected with postoperative delivery of inhaled nitric oxide. Additionally, hemodynamic parameters indirectly showed improvement in RV function while receiving inhaled milrinone and slight worsening of hemodynamics following cessation of inhaled milrinone therapy. This study was unable to determine if the delivery of inhaled milrinone caused direct or additive improvements specific to the pulmonary vasculature separate from the effects related to systemic absorption of milrinone. However, inhaled milrinone use in humans undergoing cardiac surgery has been associated with significant reductions in mean pulmonary arterial pressure, pulmonary vascular resistance, transpulmonary gradients and has been shown to facilitate separation from cardiopulmonary bypass [8-12].

### Pharmacokinetics of Inhaled Milrinone

The pharmacokinetic profile of patients receiving inhaled milrinone confirms that inhaled milrinone is systemically absorbed and that delivery of milrinone by inhaled route leads to plasma milrinone levels within an adequate range to cause systemic effect. This study presents novel findings demonstrating absorption of milrinone delivered by the inhaled route. Plasma milrinone levels remained within the accepted therapeutic range of 100-300 ng/mL [17, 18] during inhaled administration. Because of predominant renal excretion, the half-life of milrinone is increased in patients with impaired renal function. Therefore, milrinone may accumulate in patients with chronic kidney disease or perioperative cardiorenal syndrome [19]. This may cause supra-therapeutic milrinone levels and increased risk of medication related adverse events which have been associated with intravenous milrinone [20]. We did not find evidence of supra-therapeutic plasma milrinone levels or increased rates of adverse drug effects such as arrhythmias, hypotension, or drug intolerance.

### Potential Benefits of Inhaled milrinone

Inhaled pulmonary vasodilators such as nitric oxide and epoprostenol, are empirically commonly used after CF-LVAD implantation for the purpose of improving RV function by reducing RV afterload, however are expensive and cumbersome to administer. We demonstrated that inhaled milrinone can be administered using a standard IV infusion pump and delivered directly into a vibrating mesh nebulizer before entering the ventilator circuit. This makes administration of inhaled milrinone feasible in the operating room, the ICU and when transporting patients between hospital locations. Furthermore, there is no special pharmacy preparation of milrinone solution and the cost of inhaled milrinone is low at our institution. The cost per patient, for twenty-four hours of continuous inhaled milrinone, including the drug and delivery apparatus (nebulizer, syringe and IV tubing) was $165 compared to $1944 for inhaled nitric oxide. In a clinical and economic climate where hospitals and providers are increasingly pressured to deliver more cost effective and efficient care after CF-LVAD implantation, inhaled milrinone warrants further study in larger clinical trials. Future studies are currently underway to determine if the isolated effects of inhaled milrinone are beneficial for patients bridged with inotropes listed for cardiac transplantation (NCT 02077010).

### Conclusion

The data suggest that there were no significant adverse events directly related to inhaled milrinone and that clinical outcomes are similar to alternative post-operative strategies. There are substantial cost savings to inhaled milrinone over inhaled nitric oxide.

### TABLES AND TABLE LEGENDS

**Table 1 Baseline Clinical Variables**

| **Variable** | |
|---|---|
| **n=10(%)** | |
| Age, years | 56±9 |
| Male | 9(90) |
| Ischemic | 5(50) |
| BSA, kg/m² | 2.2±0.2 |
| Preoperative inotropes | 8(80)* |
| INTERMACS | 2.5±0.8 |
| RVFRS | 1.5±2.0 |
| RVSWi | 571±218 |
| Bridge to Transplant | 4(40) |
| Atrial Fibrillation | 6(60) |
| Ventricular Arrhythmia | 10(100) |
| Diabetes Mellitus | 6(60) |
| Tobacco use | 5(50) |
| History of Stroke/TIA | 3(30) |
| IABP | 2(20) |
| ICD | 10(100) |
| Sodium < 135 mg/dL | 5(50) |
| Bilirubin > 2.0 mg/dL | 4(40) |
| Albumin < 3.3 g/dL | 6(60) |
| GFR < 60mL/min | 7(70) |
| Antiarrhythmic | 5(50) |
| Loop diuretic | 10(10) |
| Hydralazine | 2(20) |
| ACE inhibitor / ARB | 6(60) |
| Aldosterone antagonist | 6(60) |
| Digoxin | 3(30) |
| Beta Blocker | 8(80) |

INTERMACS Interagency registry for mechanically assisted circulatory support, RVFRS right ventricular failure risk score, RVSWI right ventricular stroke work index - mmHg-mL/m², TIA transient ischemic attack, IABP intra-aortic balloon pump, ICD internal cardiac defibrillator, GFR estimated glomerular filtration rate, ACE angiotensin converting enzyme, ARB angiotensin receptor blocker. (* Intravenous milrinone only)

**Table 2 Perioperative Hemodynamic Comparisons**

| **Variable Compared** | **Mean Comparison (mmHg)** | | **p-Value** |
|---|---|---|---|
| **Right Atrial Pressure** | | | |
| ICU arrival vs. Mean on milrinone | 13.33 | 13.53 | 0.991 |
| Post milrinone vs. Mean on milrinone | 25.75 | 13.53 | 0.005* |
| Post milrinone vs. ICU arrival | 25.75 | 13.33 | 0.011* |
| Pre-VAD RHC vs. ICU arrival | 13.75 | 13.33 | -- |
| Pre-VAD RHC vs. Mean on milrinone | 13.75 | 13.53 | 0.841 |
| Pre-VAD RHC vs. Post milrinone | | | |
| | 13.75 | 25.75 | 0.023* |

| **Cardiac Index** | | | |
|---|---|---|---|
| ICU arrival vs. Mean on milrinone | 1.88 | 2.37 | 0.021* |
| Post milrinone vs. Mean on milrinone | 2.47 | 2.37 | 0.197 |
| Post milrinone vs. ICU arrival | 2.47 | 1.88 | 0.034* |
| Pre-VAD RHC vs. ICU arrival | 1.83 | 1.88 | 0.347 |
| Pre-VAD RHC vs. Mean on milrinone | 1.83 | 2.37 | 0.017* |
| Pre-VAD RHC vs. Post milrinone | 1.83 | 2.47 | 0.034* |

| **Cardiac Output** | | | |
|---|---|---|---|
| ICU arrival vs. Mean on milrinone | 3.99 | 5.05 | 0.035* |
| Post milrinone vs. Mean on milrinone | 5.43 | 5.05 | 0.082 |
| Post milrinone vs. ICU arrival | 5.43 | 3.99 | 0.013* |
| Pre-VAD RHC vs. ICU arrival | 3.85 | 3.99 | 0.347 |
| Pre-VAD RHC vs. Mean on milrinone | 3.85 | 5.05 | 0.028* |
| Pre-VAD RHC vs. Post milrinone | 3.85 | 5.43 | 0.012* |

| **Mean Pulmonary Artery Pressure** | | | |
|---|---|---|---|
| ICU arrival vs. Mean on milrinone | 28.5 | 25.88 | 0.912 |
| Post milrinone vs. Mean on milrinone | 27 | 25.88 | 0.575 |
| Post milrinone vs. ICU arrival | 27 | 28.5 | 0.533 |
| Pre-VAD RHC vs. ICU arrival | 39.5 | 28.5 | 0.017* |
| Pre-VAD RHC vs. Mean on milrinone | 39.5 | 25.88 | 0.017* |
| Pre-VAD RHC vs. Post milrinone | 39.5 | 27 | 0.028* |

| **Pulmonary Artery Systolic Pressure** | | | |
|---|---|---|---|
| ICU arrival vs. Mean on milrinone | 36.00 | 36.25 | 0.014* |
| Post milrinone vs. Mean on milrinone | 38.75 | 36.25 | 0.311 |
| Post milrinone vs. ICU arrival | 38.75 | 36.00 | 0.032* |
| Pre-VAD RHC vs. ICU arrival | 56.33 | 36.00 | -- |
| Pre-VAD RHC vs. Mean on milrinone | 56.33 | 36.25 | 0.014* |
| Pre-VAD RHC vs. Post milrinone | 56.33 | 38.75 | 0.032* |

| | | | |
|---|---|---|---|
| *p-value ≤0.05, ICU intensive care unit, VAD ventricular assist device, RHC right heart catheterization | | | |

**Table 3 Plasma Milrinone Pharmacokinetics**

| **Patient** | **Pre-op Creatinine** | **Post-op Creatinine** | **Post-op GFR** | **0hrs** | **1hrs** | **4hrs** | **8hrs** | **12hrs** | **24hrs** |
|---|---|---|---|---|---|---|---|---|---|
| **1** | 1.38 | 1.36 | 56 | 392.92 | 326.72 | 267.11 | 284.52 | 167.3 | 100.88 |
| **2** | 1.24 | 1.31 | 57 | 20.85 | 81.31 | 154.31 | 158.8 | * | * |
| **3** | 1.63 | 1.73 | 40 | 80.03 | 18.53 | 118.34 | 197.89 | 153.12 | 156.98 |
| **4** | 1.11 | 1.57 | 47 | 57.67 | 158.4 | 216.26 | 255.72 | 205.26 | 197.73 |
| **5** | 1.28 | 1.68 | 40 | 69.93 | 239.86 | 113.74 | 268.28 | 133.77 | * |
| **6** | 1.55 | 1.54 | 45 | NA | 115.38 | 112.71 | 115.66 | 152.27 | 117.04 |
| **7** | 1.25 | 1.43 | 50 | 65.54 | 106.94 | 114.96 | 134 | 248.58 | * |
| **8** | 3.17 | 1.96 | 34 | NA | 111.96 | 92.16 | 273.14 | 254.31 | 364.15 |
| **9** | 1.03 | 1.43 | 52 | 87.08 | 12.54 | 112.06 | 91.67 | 89.91 | 101.94 |
| **10** | 1.21 | 1.42 | 60 | 138.22 | 157.04 | 188.04 | 349.39 | * | * |
| **Mean** | 1.49 | 1.53 | 48.10 | 114.03 | 132.87 | 148.97 | 212.91 | 175.57 | 173.12 |
| **SD** | 0.62 | 0.20 | 8.45 | 117.35 | 95.39 | 57.04 | 85.52 | 56.84 | 100.79 |
| **Median** | 1.27 | 1.49 | 48.5 | 74.98 | 113.67 | 116.65 | 226.81 | 160.21 | 137.01 |

| | | | | | | | | | |
|---|---|---|---|---|---|---|---|---|---|
| * Patient extubated Patient 1 had IV milrinone stopped 6 hours before LVAD surgery; all other patients had IV milrinone stopped > 12 hours before LVAD surgery GFR - glomerular filtration rate, hrs - hours | | | | | | | | | |

**Table 4 Comparative cost analysis between Inhaled Milrinone and Inhaled Nitric Oxide per patient for 24 hours of therapy**

| **Variable** | **Unit Cost** | **# of Units** | **Total Cost** |
|---|---|---|---|
| | | | |
| Milrinone 1 mg/ml (50ml vial) | $29.27 | 3 | $87.81 |
| Aerogen^{®} Syringe | $3.50 | 5 | $17.50 |
| Aerogen^{®} Syringe Tubing Set | $6.33 | 1 | $6.33 |
| Aerogen^{®} Solo Nebulizer | $42.46 | 1 | $42.46 |
| Ventilator Expiratory Filters | $0.63 | 13 | $8.19 |
| Pharmacy Supplies | $3.00 | 1 | $3.00 |
| **Inhaled Milrinone** | | | **$165.29** |
| **Inhaled Nitric Oxide** | $81.00 | 24 | **$1,944.00** |

While the terms used herein are believed to be well understood by one of ordinary skill in the art, the definitions set forth herein are provided to facilitate explanation of the presently-disclosed subject matter.

Unless defined otherwise, all technical and scientific terms used herein have the same meaning as commonly understood by one of ordinary skill in the art to which the presently-disclosed subject matter belongs. Although any methods, devices, and materials similar or equivalent to those described herein can be used in the practice or testing of the presently-disclosed subject matter, representative methods, devices, and materials are now described.

Following long-standing patent law convention, the terms "a", "an", and "the" refer to "one or more" when used in this application, including the claims. Thus, for example, reference to "a composition" includes a plurality of such compositions, and so forth.

Unless otherwise indicated, all numbers expressing quantities of ingredients, properties such as reaction conditions, and so forth used in the specification and claims are to be understood as being modified in all instances by the term "about". Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of mass, weight, time, volume, concentration or percentage is meant to encompass variations of in some embodiments ±50%, in some embodiments ±40%, in some embodiments ±30%, in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

As used herein, ranges can be expressed as from "about" one particular value, and/or to "about" another particular value. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

### REFERENCES

1. Kirklin JK, Naftel DC, Pagani FD, Kormos RL, Stevenson LW, Blume ED, et al. Sixth INTERMACS annual report: A 10,000-patient database. J Heart Lung Transplant. 2014 Jun;33(6):555-64.
2. Kormos RL, Teuteberg JJ, Pagani FD, Russell SD, John R, Miller LW, et al. Right ventricular failure in patients with the HeartMate II continuous-flow left ventricular assist device: Incidence, risk factors, and effect on outcomes. J Thorac Cardiovasc Surg. 2010 May;139(5): 1316-24.
3. Miller LW, Lietz K. Candidate selection for long-term left ventricular assist device therapy for refractory heart failure. J Heart Lung Transplant. 2006 07;25(7):756-64.
4. John R, Pagani FD, Naka Y, Boyle A, Conte JV, Russell SD, et al. Post-cardiac transplant survival after support with a continuous-flow left ventricular assist device: Impact of duration of left ventricular assist device support and other variables. J Thorac Cardiovasc Surg. 2010 07;140(1):174-81.
5. Potapov E, Meyer D, Swaminathan M, Ramsay M, El Banayosy A, Diehl C, et al. Inhaled nitric oxide after left ventricular assist device implantation: A prospective, randomized, double-blind, multicenter, placebo-controlled trial. J Heart Lung Transplant. 2011 Aug;30(8):870-8.
6. Hasenfuss G, Teerlink JR. Cardiac inotropes: Current agents and future directions. Eur Heart J. 2011 Aug;32(15): 1838-45.
7. Felker GM, Benza RL, Chandler AB, Leimberger JD, Cuffe MS, Califf RM, et al. Heart failure etiology and response to milrinone in decompensated heart failure: Results from the OPTIME-CHF study. J Am Coll Cardiol. 2003 Mar 19;41(6):997-1003.
8. Haraldsson sA, Kieler-Jensen N, Ricksten SE. The additive pulmonary vasodilatory effects of inhaled prostacyclin and inhaled milrinone in postcardiac surgical patients with pulmonary hypertension. Anesth Analg. 2001 Dec;93(6):1439,45, table of contents.
9. Sablotzki A, Starzmann W, Scheubel R, Grond S, Czeslick EG. Selective pulmonary vasodilation with inhaled aerosolized milrinone in heart transplant candidates. Can J Anaesth. 2005 12;52(10):1076-82.
10. Wang H, Gong M, Zhou B, Dai A. Comparison of inhaled and intravenous milrinone in patients with pulmonary hypertension undergoing mitral valve surgery. Adv Ther. 2009 Apr;26(4):462-8.
11. Singh R, Choudhury M, Saxena A, Kapoor PM, Juneja R, Kiran U. Inhaled nitroglycerin versus inhaled milrinone in children with congenital heart disease suffering from pulmonary artery hypertension. J Cardiothorac Vasc Anesth. 2010 Oct;24(5):797-801.
12. Buckley MS, Feldman JP. Nebulized milrinone use in a pulmonary hypertensive crisis. Pharmacotherapy. 2007 12;27(12):1763-6.
13. Fitzpatrick JR,3rd, Frederick JR, Hsu VM, Kozin ED, O'Hara ML, Howell E, et al. Risk score derived from pre-operative data analysis predicts the need for biventricular mechanical circulatory support. J Heart Lung Transplant. 2008 Dec;27(12):1286-92.
14. Nguyen AQ, Theoret Y, Chen C, Denault A, Varin F. High performance liquid chromatography using UV detection for the quantification of milrinone in plasma: Improved sensitivity for inhalation. J Chromatogr B Analyt Technol Biomed Life Sci. 2009 Mar 1;877(7):657-60.
15. Haglund NA, Luethge M, Duryee M, Hunter C, Alnouti Y, Corcoran T, Beck J, Maltais S, Lenihan D, Um J, Dumitru I. Pre-Clinical Testing of Aerosolized Inhaled Milrinone Using a Vibrating Mesh Nebulizer. ; April 2013. S252-S253 p.
16. Matthews JC, Koelling TM, Pagani FD, Aaronson KD. The right ventricular failure risk score a pre-operative tool for assessing the risk of right ventricular failure in left ventricular assist device candidates. J Am Coll Cardiol. 2008 Jun 3;51(22):2163-72.
17. Jaski BE, Fifer MA, Wright RF, Braunwald E, Colucci WS. Positive inotropic and vasodilator actions of milrinone in patients with severe congestive heart failure. dose-response relationships and comparison to nitroprusside. J Clin Invest. 1985 Feb;75(2):643-9.
18. Vazir A, Leaver N, Lyster H, Alexanian A, Wilton P, Banner NR. Is monitoring milrinone therapy useful in advanced heart failure? Int J Cardiol. 2011 Jun 16;149(3):380-1.
19. Charisopoulou D, Leaver N, Banner NR. Milrinone in advanced heart failure: Dose and therapeutic monitor outside intensive care unit. Angiology. 2014 Apr;65(4):343-9.
20. Cox ZL, Calcutt MW, Morrison TB, Akers WS, Davis MB, Lenihan DJ. Elevation of plasma milrinone concentrations in stage D heart failure associated with renal dysfunction. J Cardiovasc Pharmacol Ther. 2013 Sep;18(5):433-8.

## Claims

1. A pharmaceutical composition comprising a pharmaceutically acceptable carrier, an excipient, a surfactant; and milrinone at a concentration of 10 to 17 mg/mL, at a pH of about 2.6 to about 3.4 for use in a method for treating heart failure, in a subject in need thereof, comprising:
providing said pharmaceutical composition; and
nebulizing said pharmaceutical composition and administrating said nebulized pharmaceutical composition by inhalation, e.g. every about 4 to about 12 hours.

2. The pharmaceutical composition for use according to claim 1, wherein the heart failure is following cardiac surgery.

3. The pharmaceutical composition for use according to claim 1, wherein the subject is diagnosed as having stage D ambulatory heart failure or right ventricular failure.

4. The pharmaceutical composition for use according to claim 1, wherein the pH is from about 3.0 to about 3.2.

5. The pharmaceutical composition for use according to claim 1, wherein the pharmaceutical composition is provided in an airtight container together with a propellant.

6. The pharmaceutical composition for use according to claim 1, wherein the pharmaceutical composition is administered by operating a nebulizer, such as a jet nebulizer, an ultrasonic nebulizer or a vibrating mesh nebulizer, which nebulizer includes a container for housing a volume of the composition, such as about 1 mL to about 100 mL of the composition.

7. A pharmaceutical composition comprising sterile water, lactic acid; and milrinone at a concentration of 10 to 17 mg/mL, at a pH of about 2.6 to about 3.4 for use in a method for treating heart failure, in a subject in need thereof, comprising:
providing said pharmaceutical composition; and
nebulizing said pharmaceutical composition and administrating said nebulized pharmaceutical composition by inhalation, e.g. every about 4 to about 12 hours.

8. The pharmaceutical composition for use according to claim 7, wherein the heart failure is following cardiac surgery.

9. The pharmaceutical composition for use according to claim 7, wherein the subject is diagnosed as having stage D ambulatory heart failure or right ventricular failure.

10. The pharmaceutical composition for use according to claim 7, wherein the pH is from about 3.0 to about 3.2.

11. The pharmaceutical composition for use according to claim 7, wherein the pharmaceutical composition is provided in an airtight container together with a propellant.

12. The pharmaceutical composition for use according to claim 7, wherein the pharmaceutical composition is administered by operating a nebulizer, such as a jet nebulizer, an ultrasonic nebulizer or a vibrating mesh nebulizer, which nebulizer includes a container for housing a volume of the composition, such as about 1 mL to about 100 mL of the composition.

## Patentansprüche

1. Pharmazeutische Zusammensetzung umfassend einen pharmazeutisch akzeptablen Träger, einen Trägerstoff, ein Tensid und Milrinon in einer Konzentration von 10 bis 17 mg/ml, bei einem pH-Wert von etwa 2,6 bis etwa 3,4, zur Verwendung bei einem Verfahren für die Behandlung von Herzinsuffizienz bei einem Subjekt, das deren bedarf, umfassend:
Bereitstellen der pharmazeutischen Zusammensetzung; und
Vernebeln der pharmazeutischen Zusammensetzung und Verabreichen der vernebelten pharmazeutischen Zusammensetzung durch Inhalation, z.B. etwa alle 4 bis 12 Stunden.

2. Pharmazeutische Zusammensetzung zur Verwendung nach Anspruch 1, wobei die Herzinsuffizienz auf eine Herzchirurgie hin auftritt.

3. Pharmazeutische Zusammensetzung zur Verwendung nach Anspruch 1, wobei das Subjekt als ambulatorische Herzinsuffizienz der Stufe D oder Versagen des rechten Ventrikels aufweisend diagnostiziert ist.

4. Pharmazeutische Zusammensetzung zur Verwendung nach Anspruch 1, wobei der pH-Wert etwa 3,0 bis etwa 3,2 beträgt.

5. Pharmazeutische Zusammensetzung zur Verwendung nach Anspruch 1, wobei die pharmazeutische Zusammensetzung in einem luftdichten Behälter zusammen mit einem Treibmittel bereitgestellt wird.

6. Pharmazeutische Zusammensetzung zur Verwendung nach Anspruch 1, wobei die pharmazeutische Zusammensetzung durch Betätigen eine Verneblers, wie beispielsweise eines Düsenverneblers, eines Ultraschallverneblers oder eines vibrierenden Maschenverneblers verabreicht wird, welcher Vernebler einen Behälter zum Unterbringen eines Volumens der Zusammensetzung, wie beispielsweise etwa 1 ml bis etwa 100 ml der Zusammensetzung, umfasst.

7. Pharmazeutische Zusammensetzung umfassend steriles Wasser, Milchsäure und Milrinon in einer Konzentration von 10 bis 17 mg/ml, bei einem pH-Wert von etwa 2,6 bis etwa 3,4, zur Verwendung bei einem Verfahren für die Behandlung von Herzinsuffizienz bei einem Subjekt, das deren bedarf, umfassend:
Bereitstellen der pharmazeutischen Zusammensetzung; und
Vernebeln der pharmazeutischen Zusammensetzung und Verabreichen der vernebelten pharmazeutischen Zusammensetzung durch Inhalation, z.B. etwa alle 4 bis 12 Stunden.

8. Pharmazeutische Zusammensetzung zur Verwendung nach Anspruch 7, wobei die Herzinsuffizienz auf Herzchirurgie hin auftritt.

9. Pharmazeutische Zusammensetzung zur Verwendung nach Anspruch 7, wobei das Subjekt als ambulatorische Herzinsuffizienz der Stufe D oder Versagen des rechten Ventrikels aufweisend diagnostiziert ist.

10. Pharmazeutische Zusammensetzung zur Verwendung nach Anspruch 7, wobei der pH-Wert etwa 3,0 bis etwa 3,2 beträgt.

11. Pharmazeutische Zusammensetzung zur Verwendung nach Anspruch 7, wobei die pharmazeutische Zusammensetzung in einem luftdichten Behälter zusammen mit einem Treibmittel bereitgestellt wird.

12. Pharmazeutische Zusammensetzung zur Verwendung nach Anspruch 7, wobei die pharmazeutische Zusammensetzung durch Betätigen eine Verneblers, beispielsweise eines Düsenverneblers, eines Ultraschallverneblers oder eines vibrierenden Maschenverneblers verabreicht wird, welcher Vernebler einen Behälter zum Unterbringen eines Volumens der Zusammensetzung, wie beispielsweise etwa 1 ml bis etwa 100 ml der Zusammensetzung, umfasst.

## Revendications

1. Composition pharmaceutique comprenant un véhicule, un excipient, un tensioactif acceptable sur le plan pharmaceutique ; et de la milrinone à une concentration comprise entre 10 et 17 mg/mL, à un pH compris entre environ 2,6 et 3,4 destinée à être utilisée dans un procédé destiné au traitement de l'insuffisance cardiaque, chez un sujet en ayant besoin, consistant à :
fournir la composition pharmaceutique ; et
nébuliser ladite composition pharmaceutique et administrer ladite composition pharmaceutique nébulisée par inhalation, par exemple toutes les 4 à 12 heures environ.

2. Composition pharmaceutique destinée à être utilisée selon la revendication 1, dans laquelle l'insuffisance cardiaque fait suite à une chirurgie cardiaque.

3. Composition pharmaceutique destinée à être utilisée selon la revendication 1, dans laquelle le sujet est diagnostiqué comme ayant une insuffisance cardiaque ambulatoire de stade D ou une insuffisance ventriculaire droite.

4. Composition pharmaceutique destinée à être utilisée selon la revendication 1, dans laquelle le pH est compris entre environ 3,0 et environ 3,2.

5. Composition pharmaceutique destinée à être utilisée selon la revendication 1, dans laquelle la composition pharmaceutique est disposée dans un récipient hermétique à l'air avec un propulseur.

6. Composition pharmaceutique destinée à être utilisée selon la revendication 1, dans laquelle la composition pharmaceutique est administrée en faisant fonctionner un nébuliseur, tel qu'un nébuliseur à jet, un nébuliseur à ultrasons ou un nébuliseur à tamis vibrant, lequel nébuliseur comprend un récipient destiné à contenir un volume de la composition, tel qu'environ 1 mL à environ 100 mL de la composition.

7. Composition pharmaceutique comprenant de l'eau stérile, de l'acide lactique ; et de la milrinone à une concentration comprise entre 10 et 17 mg/mL, à un pH d'environ 2,6 à environ 3,4 destinée à être utilisé dans un procédé destiné au traitement de l'insuffisance cardiaque, chez un sujet en ayant besoin, consistant à :
fournir ladite composition pharmaceutique ; et
nébuliser ladite composition pharmaceutique et administrer ladite composition pharmaceutique nébulisée par inhalation, par exemple toutes les 4 à 12 heures environ.

8. Composition pharmaceutique destinée à être utilisée selon la revendication 7, dans laquelle l'insuffisance cardiaque fait suite à une chirurgie cardiaque.

9. Composition pharmaceutique destinée à être utilisée selon la revendication 7, dans laquelle le sujet est diagnostiqué comme ayant une insuffisance cardiaque ambulatoire de stade D ou une insuffisance ventriculaire droite.

10. Composition pharmaceutique destinée à être utilisée selon la revendication 7, dans laquelle le pH est compris entre environ 3,0 et environ 3,2.

11. Composition pharmaceutique destinée à être utilisée selon la revendication 7, dans laquelle la composition pharmaceutique est disposée dans un récipient hermétique à l'air avec un propulseur.

12. Composition pharmaceutique destinée à être utilisée selon la revendication 7, dans laquelle la composition pharmaceutique est administrée en faisant fonctionner un nébuliseur, tel qu'un nébuliseur à jet, un nébuliseur à ultrasons ou un nébuliseur à tamis vibrant, lequel nébuliseur comprend un récipient destiné à contenir un volume de la composition, tel qu'environ 1 mL à environ 100 mL de la composition.
